# FASCICULE DE BREVET EUROPEEN

(11) **EP 2 870 234 B1**
(45) Date de publication et mention de la délivrance du brevet: **23.08.2017**
(21) Numéro de dépôt: 13734435.4
(22) Date de dépôt: 09.07.2013
(51) Int. Cl.: C12N 1/20, A61K 35/74, C12R 1/225

(54) **NOUVELLE SOUCHE DE LACTOBACILLUS CRISPATUS**
NEUARTIGER STAMM VON LACTOBACILLUS CRISPATUS
NOVEL STRAIN OF LACTOBACILLUS CRISPATUS

(30) Priorité: 09.07.2012 FR 1256569
(43) Date de publication de la demande: 13.05.2015
(73) Titulaire: S.P.M.D, 75010 Paris (FR)
(72) Inventeur: NIVOLIEZ, Adrien, F-15130 Yolet (FR)
(74) Mandataire: Regimbeau
(86) Numéro de dépôt international: PCT/EP2013/064424
(87) Numéro de publication internationale: WO 2014/009330

(56) Documents cités:
- EP-A2- 0 949 330
- WO-A1-03/038068
- US-A1- 2010 151 026
- J. G. WYLIE ET AL: "IDENTITY AND GLYCOGEN-FERMENTING ABILITY OF LACTOBACILLI ISOLATED FROM THE VAGINA OF PREGNANT WOMEN", JOURNAL OF MEDICAL MICROBIOLOGY, vol. 2, no. 3, 1 août 1969 (1969-08-01), pages 363-366, XP055056992, ISSN: 0022-2615, DOI: 10.1099/00222615-2-3-363
- MARTIN REBECA ET AL: "Characterization of indigenous vaginal lactobacilli from healthy women as probiotic candidates", INTERNATIONAL MICROBIOLOGY, vol. 11, no. 4, décembre 2009 (2009-12), pages 261-266, XP002694210, ISSN: 1139-6709 cité dans la demande
- R. W. HYMAN ET AL: "Microbes on the human vaginal epithelium", PROCEEDINGS OF THE NATIONAL ACADEMY OF SCIENCES, vol. 102, no. 22, 31 mai 2005 (2005-05-31) , pages 7952-7957, XP055057092, ISSN: 0027-8424, DOI: 10.1073/pnas.0503236102 -& DATABASE EMBL [Online] 3 juin 2005 (2005-06-03), "Uncultured bacterium clone rRNA245 16S ribosomal RNA gene, partial sequence.", XP002694211, extrait de EBI accession no. EMBL:AY959018 Database accession no. AY959018 -& DATABASE EMBL [Online] 3 juin 2005 (2005-06-03), "Uncultured bacterium clone rRNA376 16S ribosomal RNA gene, partial sequence.", XP002694212, extrait de EBI accession no. EMBL:AY959149 Database accession no. AY959149

## Description

### DOMAINE DE L'INVENTION

La présente invention concerne une nouvelle souche spécifique de *Lactobacillus crispatus,* isolée à partir d'échantillons tissulaires sains, et toute souche présentant les mêmes caractéristiques, ainsi que des compositions les comprenant.

Chez une femme saine, la flore urogénitale comprend près de 50 espèces différentes de microorganismes. Parmi ces microorganismes, 95% de la population sont constitués de diverses souches de lactobacilles, dont le « bacille de Döderlein ». Ces lactobacilles jouent un rôle de protection contre les pathogènes par divers mécanismes : production de peroxyde d'hydrogène, production d'acide lactique, production de bactériocines, inhibition de l'adhésion et de l'expansion des pathogènes. En particulier, ces lactobacilles maintiennent un pH acide par la production d'acide lactique à partir du glycogène présent dans le mucus vaginal. Ainsi, la croissance de nombreux pathogènes de la flore vaginale, tels que *Gardnerella vaginalis, Prevotella bivia, Neisseria gonorrhoeae,* les mycoplasmes, *Mobiluncus* et surtout *Candida albicans,* est inhibée.

La flore vaginale normale est ainsi principalement composée de lactobacilles formant un biofîlm protecteur à la surface de la muqueuse. Les lactobacilles les plus fréquemment observés dans le vagin sont notamment *Lactobacillus crispatus, Lactobacillus jensenii, Lactobacillus vaginalis, Lactobacillus iners* et *Lactobacillus gasseri.*

La candidose vulvovaginale touche 70-75% des femmes en période d'activité génitale au moins une fois dans leur vie ; environ 40-50% d'entre elles présenteront un deuxième épisode. L'incidence de la candidose vulvovaginale récidivante (définie par la survenue d'au moins 4 épisodes par an, dont deux prouvés par l'examen mycologique) a été estimée à 5-8%. Cette affection bénigne a un impact très négatif sur la qualité de vie des patientes et engendre des dépenses de santé importantes. Traiter une telle pathologie est difficile du fait de la pathogénèse multifactorielle de cette affection.

Le traitement d'entretien traditionnel par antifongique oral ou vaginal doit être de 6 mois au moins, mais le taux de rechutes reste élevé avec 60-70% des femmes présentant une récidive dans les deux mois suivant l'arrêt du traitement. De plus, les effets secondaires des antifongiques sont fréquents et leur utilisation au long cours peut favoriser la survenue d'une vaginose bactérienne.

La vaginose bactérienne est due à un déséquilibre qualitatif et quantitatif de la flore vaginale normale pouvant aboutir à une disparition quasi complète des lactobacilles au profit de la flore anaérobie et aussi à l'émergence de germes tels que *Gardnerella vaginalis* et *Atopobium vaginae* impliqués dans la vaginose bactérienne.

La vaginose bactérienne est une des infections vaginales les plus fréquentes avec un taux de fréquence de 10 à 15. Cette pathologie bénigne chez la femme peut être grave pendant la grossesse, car pouvant entraîner un accouchement prématuré, des petits poids de naissance, ainsi que des avortements spontanés.

La vaginose bactérienne ainsi que les autres déséquilibres de la microflore vaginale sont communément traités par antibiothérapie. Ce traitement présente les inconvénients habituels des traitements antibiotiques et s'avère de moins en moins efficace. En outre, il vise à éliminer la flore pathogène mais détruit également la flore normale bénéfique.

### ETAT DE LA TECHNIQUE

L'administration de lactobacilles bénéfiques, par voie orale ou vaginale, a été décrite pour promouvoir la santé vaginale et pour traiter les infections génitales. En particulier, les demandes de brevet WO 84/04675, WO 2000/035465, US 2002/0044926 et WO 2006/045475 décrivent l'administration par voie orale ou vaginale de bactéries lactiques pour promouvoir la santé vaginale et pour prévenir les récidives de la candidose vulvovaginale.

Les lactobacilles préférés sont *Lactobacillus rhamnosus* et le bacille de Döderlein *(Lactobacillus acidophilus vaginalis).* Les brevets US 6.093.394, US 6.468.526 et US 7.807.440, ainsi que la demande de brevet US 2010/0151026, décrivent l'administration de souches spécifiques de *Lactobacillus crispatus.*

Parmi ces souches, on pourra citer les souches de *Lactobacillus crispatus* suivantes :
- Les souches de *Lactobacillus crispatus* déposées auprès de la DSMZ (Allemagne) sous les numéros de dépôt DSM 16735, 16736, 16738, 16739, 16740, 16741, 16742 et 16743, telles que décrites dans la demande de brevet EP 1 824 500 ;ces souches sont capables de coloniser les muqueuses vaginales, et ainsi de favoriser la santé vaginale ; elles ont également de bonnes propriétés d'adhésion aux cellules épithéliales ;
- Les souches de *Lactobacillus crispatus* déposées auprès de la CNCM (France) sous les numéros de dépôt I-3483, I-3484 et I-3486, telles que décrites dans la demande de brevet EP 1 812 023 ; ces souches ont été sélectionnées pour leur capacité d'adhérence aux cellules épithéliales ; elles présentent une bonne résistance aux pH acides de l'environnement gastrique, ainsi qu'un effet anti-pathogène avéré ;
- La souche de *Lactobacillus crispatus* déposée auprès de la BCCM/LMG (Belgique) sous le numéro de dépôt P-20558 telle que décrite dans le brevet EP 1 427 808 B1 ; cette souche est utilisée en combinaison avec d'autres souches lactiques, pour être administrée à l'aide d'un tampon, cette souche présentant la capacité de pouvoir coloniser la cavité vaginale, y compris pendant la menstruation ; et
- La souche de *Lactobacillus crispatus* déposée auprès de l'ATCC (USA) sous le numéro de dépôt 202225, telle que décrite dans le brevet EP 1 011 721 B1 ; cette souche présente une bonne adhérence aux cellules épithéliales, une viabilité de longue durée, notamment en présence de la matrice de conservation décrite dans ce document, ainsi qu'une bonne production d'acide lactique et de H₂O₂.
- La souche de *Lactobacillus crispatus P-17631* telle que décrite dans la demande de brevet EP 949 330 est capable de fermenter le glycogène, et d'inhiber la croissance des *Candida.*

La plupart de ces souches probiotiques, isolées à partir de prélèvements vaginaux de femmes saines, ne sont pas capables de survivre dans un milieu présentant un pH acide, ni de survivre à un contact prolongé avec de la bile.

Le pH vaginal est un paramètre qui varie de manière significative durant la vie de la femme : le pH physiologique est de 7 chez les filles prépubères, descend jusqu'à 3,8 chez les femmes pubères, et est supérieur à 5,5 pendant la ménopause, en absence de traitement substitutif hormonal. Ces variations sont liées à de multiples facteurs tels que la présence d'oestrogènes et la colonisation par les lactobacilles.

Il est donc important pour les souches probiotiques administrées de façon exogène de pouvoir résister aux pH acides physiologiques de la cavité vaginale, et de plus de pouvoir résister au pH de la cavité gastrique lors d'une administration par voie orale.

Par ailleurs, nombre de ces souches ne sont pas capables d'utiliser le glycogène comme source de carbone fermentable (Martin R. et al., 2008). Or, cette propriété est avantageuse, car il est fréquent que la muqueuse vaginale soit dépourvue de glucose, et le maintien de la production d'acide lactique par la souche probiotique ne peut se faire qu'en présence d'une source de carbone fermentable.

### EXPOSE DE L'INVENTION

La présente invention est relative à une nouvelle souche isolée de *Lactobacillus crispatus,* identifiée *IP174178,* déposée à la CNCM en date du 22 Juin 2012 sous le numéro d'accès CNCM I-4646.

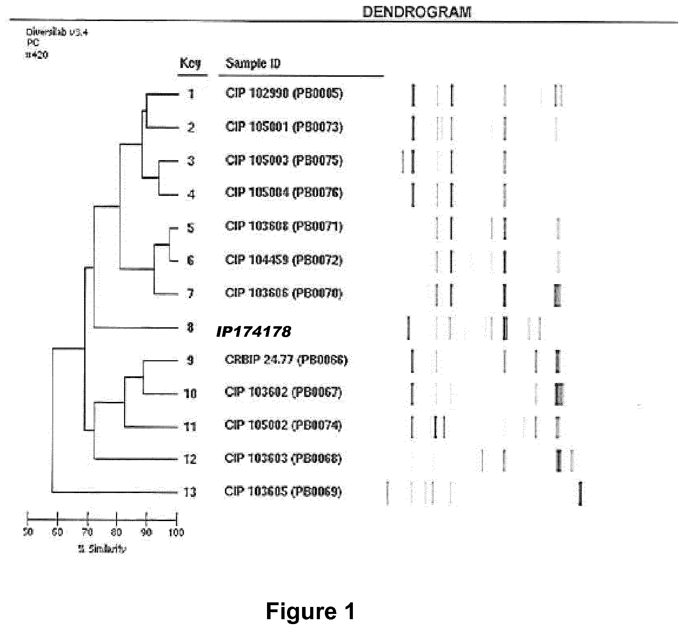

Cette nouvelle souche *IP174178* présente les caractéristiques structurelles suivantes :
- elle pousse en conditions aérobie-anaérobie;
- son profil rep-PCR est présenté en figure 1 ;
- la séquence de son ADN ribosomal 16S est présentée en SEQ ID N°1.

Ses caractéristiques fonctionnelles principales sont :
- son fort taux de production de H₂O₂ et d'acide lactique (plus de 7 g/litre) ;
- sa capacité à croître sur du glycogène comme seul substrat carboné ;
- sa résistance à la bile ;
- sa résistance à des pH acides (notamment à des pH compris entre 2,5 et 4) ;
- sa capacité à inhiber le développement de pathogènes de la zone urogénitale.

La souche *Lactobacillus crispatus IP174178* présente en particulier une résistance aux pH acides.

Plus particulièrement, la souche *Lactobacillus crispatus IP174178* présente une résistance à la bile et aux pH acides.

Plus particulièrement, la souche *Lactobacillus crispatus IP174178* présente une résistance à la bile et aux pH acides, et est capable de métaboliser le glycogène.

L'invention est également relative à une composition pharmaceutique comprenant au moins 10⁹ UFC de cette souche *Lactobacillus crispatus IP174178,* seule ou en combinaison avec d'autres souches probiotiques ou d'autres principes actifs.

L'invention est également relative à l'utilisation de cette souche *IP174178* pour la prévention et le traitement d'infections génitales et urogénitales, récidivantes ou non.

### DESCRIPTION DETAILLEE DE L'INVENTION

La présente invention est relative à une nouvelle souche isolée *Lactobacillus crispatus identifiée IP174178, et* déposée à la CNCM sous le numéro d'accès I-4646. Le terme « lactobacilles » désigne l'ensemble des bactéries lactobacilles, bactéries à gram positif, immobiles, de formes et dimensions variables, et anaérobies facultatives. La plupart des lactobacilles convertissent le lactose et d'autres sucres simples en acide lactique. On les trouve dans le vagin et le tractus gastro-intestinal et ils constituent un élément important de la flore intestinale et vaginale.

Le genre « *Lactobacillus crispatus* » est une des espèces prédominantes de la flore vaginale en Europe, aux Etats-Unis et au Japon (Antonio, MAD et al., 1999).

La souche isolée *Lactobacillus crispatus IP174178* présente les caractéristiques fonctionnelles suivantes :
- Forte production d'H₂O₂ et d'acide lactique (plus de 7 g/litre);
- Capacité à croître sur du glycogène, substrat endogène secrété par la muqueuse vaginale ;
- Résistance aux pH acides ainsi qu'à la bile ;
- Capacité à inhiber le développement de pathogènes ;
- Adhésion aux cellules épithéliales vaginales.

Cette capacité à croître sur du glycogène est relativement rare, et revêt un intérêt particulier pour des souches destinées à se développer dans le tractus génital, où le glucose est souvent absent, et où la seule source de carbone est le glycogène. La production d'acide lactique par cette souche est donc maintenue même en absence de glucose (voir tableau 5).

La capacité à résister à des pH acides est une caractéristique très intéressante, car elle permet à la souche selon l'invention d'une part de résister au pH gastrique si celle-ci est administrée par voie orale, d'autre part de se reproduire dans le tractus génital féminin même en cas de sécrétions anormalement acides.

La capacité à résister à la bile permet d'envisager une administration de cette souche par voie orale, et non uniquement en administration vaginale.

L'invention est également relative à une composition pharmaceutique comprenant la souche *Lactobacillus crispatus IP174178.*

Dans la composition selon l'invention, le nombre de bactéries par gramme de composition est de préférence supérieur à 10⁸, et est notamment compris entre 10⁸ et 10¹⁰. Selon un aspect particulier de l'invention, la composition comprend au moins 10⁹ UFC de la souche de *Lactobacillus crispatus.* Le terme « UFC » signifie « Unité Formant Colonie » et est l'unité de mesure généralement reconnue par l'homme du métier pour quantifier les bactéries capables de fonder une colonie.

De préférence, la souche de *Lactobacillus crispatus* est sous forme lyophilisée. La souche peut être le seul élément lyophilisé de la composition, mais de préférence la souche est lyophilisée dans un milieu comprenant des constituants additionnels, qui seront ajoutés avant ou après l'étape de lyophilisation.

Selon un autre aspect particulier de l'invention, la composition pharmaceutique comprend de plus une matrice de conservation et/ou des excipients bien connus de l'homme du métier, et optionnellement d'autres principes actifs ayant une action complémentaire.

Selon un aspect particulier de l'invention, la composition est sous forme de poudre, qui peut être placée dans une capsule, ou qui peut être intégrée à toute sorte de véhicule approprié tel qu'une crème ou une composition nutraceutique.

En particulier, cette composition peut comprendre les principes actifs suivants : des hormones et/ou des agents anti-inflammatoires et/ou des agents bactéricides et/ou des agents anti-fongiques. L'homme du métier saura déterminer quels sont les principes actifs qui peuvent être avantageusement couplés à la souche *Lactobacillus crispatus IP174178.*

Selon un aspect préféré de l'invention, la composition comprend du thiosulfate de sodium, de préférence dans une concentration d'au moins 100 mg/gramme de poudre comprenant entre 10⁸ et 10¹⁰ UFC de *Lactobacillus crispatus IP174178.* Les exemples ci-après illustrent une composition dénommée « Formulation A » qui comprend 113 g/l de thiosulfate de sodium avant lyophilisation, et au final 230 mg de thiosulfate de sodium/g de poudre La présence de thiosulfate de sodium permet de potentialiser l'effet anti-pathogène de la souche *IP174178* (voir tableau 6C).

La composition selon l'invention peut également comprendre d'autres souches de Lactobacillus.

Selon l'invention, la composition pharmaceutique peut être formulée pour une administration par voie vaginale ou par voie orale. En particulier, les bactéries destinées à être administrées par voie orale ont été testées pour leur résistance à la bile, leur résistance aux pH acides et pour leur capacité de croissance en présence de bile. La souche *IP174178* de l'invention présente ces caractéristiques lui permettant d'être administrée par voie orale.

Pour des compositions pharmaceutiques pour administration orale, les formulations appropriées sont notamment les comprimés, les gélules, les poudres, les granules, les solutions et les suspensions orales.

Pour des compositions pharmaceutiques pour administration vaginale, les formulations appropriées sont notamment les crèmes, les gélules, ainsi que les suppositoires, ovules et tampons vaginaux.

La composition selon l'invention est en particulier destinée à être utilisée dans le traitement d'infections génitales et urogénitales, telles que les vaginoses et les candidoses.

On entend par 'vaginose' un déséquilibre de la flore microbienne du vagin. Elle se caractérise par la disparition des lactobacilles et la multiplication de germes pathogènes tels que listés ci-dessus.

On entend par 'candidose' une infection vaginale causée par des levures du genre Candida.

Ces infections sont liées à la présence de pathogènes, en particulier appartenant à la famille des *Candida albicans, des Gardnerella vaginalis,* des mycoplasmes ou des *Mobiluncus.*

*Candida albicans* est l'espèce de levure la plus importante et la plus connue du genre Candida. Elle provoque des infections fongiques (candidose, aussi appelée candidiase) essentiellement au niveau des muqueuses digestives et génitales.

*Gardnerella vaginalis* est une bactérie se présentant comme des bâtonnets pléomorphes ou des coccobacilles. C'est une bactérie retrouvée fréquemment en cas de vaginose (vaginite non spécifique), soit comme seul germe pathogène soit en association avec d'autres bactéries. Elle produit une toxine perforante qui n'affecte que les cellules humaines.

Le terme mycoplasmes désigne une famille de plus de 100 espèces de bactéries, qui sont insensibles aux familles antibiotiques ciblant les parois cellulaires (pénicilline ou bêtalactames). En particulier, *Mycoplasma genitalium* est responsable d'infections génitales et urogénitales (urétrite, cervicite, vaginite, salpingite) et de problèmes de stérilité.

*Mobiluncus* est une bactérie gram positive, anaérobie, souvent retrouvée associée à *Gardnerella vaginalis* dans des vaginoses bactériennes.

L'invention est également relative à une composition nutraceutique comprenant la souche *Lactobacillus crispatus* IP174178. Une telle composition nutraceutique se présentera sous la forme d'une composition alimentaire ou d'un complément alimentaire.

Une composition alimentaire sera composée de la souche *Lactobacillus crispatus IP174178,* lyophilisée de préférence, mélangée à des produits alimentaires tels que des produits laitiers, des matières grasses, des produits à base de céréales, des confiseries, des boissons.

Le complément alimentaire peut en particulier se présenter sous forme de gélules ou de capsules molles en gélatine ou matière végétale, dans le cadre de la présente invention.

L'invention se rapporte également à un dispositif médical comprenant l'une des compositions telles que décrites ci-dessus, ce dispositif médical pouvant être en particulier un tampon vaginal, une spatule, une serviette hygiénique, un savon à usage intime, etc.

L'invention se rapporte également à l'utilisation de la souche isolée *Lactobacillus crispatus IP174178,* pour la prévention et le traitement d'infections génitales et urogénitales telles que les vaginoses et les candidoses.

Les modes d'administration, les posologies et les formes galéniques optimales des compositions selon l'invention peuvent être déterminés selon les critères généralement pris en compte dans l'établissement d'un traitement pharmaceutique adapté à un patient, comme par exemple l'âge ou le poids corporel du patient, la gravité de son état général, la tolérance au traitement, les effets secondaires constatés. La souche isolée *Lactobacillus crispatus IP174178* sera utilisée à la fois en administration locale (vaginale) ou en administration orale, les deux types d'administration étant envisagés seuls ou en combinaison.

Selon un autre aspect de l'invention, la souche isolée *Lactobacillus crispatus IP174178* est utilisée pour maintenir une flore vaginale bénéfique chez une femme ne présentant pas d'infections.

Selon encore un autre aspect de l'invention, la souche isolée *Lactobacillus crispatus IP174178* est utilisée pour la restauration d'une flore vaginale bénéfique suite à un traitement antibiotique.

L'invention se rapporte donc à une composition pharmaceutique comprenant la souche isolée *Lactobacillus crispatus IP174178,* pour son utilisation dans le maintien ou la restauration d'une flore vaginale bénéfique.

### DESCRIPTION DES FIGURES

Figure 1 : Représentation graphique sous forme de dendrogramme de la comparaison des profils génotypiques par rep-PCR.
Figure 2 : Représentation graphique sous forme de matrice de similarité de la comparaison des profils génotypiques par rep-PCR.
Figure 3 : Test d'adhérence de la souche IP174178 à des cellules épithéliales vaginales.

### EXEMPLES

### Exemple 1- isolement et sélection de la souche L. crispatus IP174178

Sept tests ont été appliqués à 10 souches isolées à partir de femmes saines, et présélectionnées pour leur capacité à produire du peroxyde d'hydrogène (H₂O₂). Ces tests sont : la croissance en milieu MRS (Man JC et al., 1960) modifié, la capacité à produire de l'H₂O₂, la capacité à produire de l'acide lactique, l'adhésion à des cellules de lignée vaginale, la capacité à inhiber certains pathogènes génitaux (méthode des disques inhibés) et la résistance au métronidazole.

A la suite de ces premiers tests, trois souches identifiées comme particulièrement intéressantes, notamment pour leur importante production d'H₂O₂ et d'acide lactique, ont fait l'objet d'un test de croissance en présence de glycogène, la souche *IP174178* ayant seule la capacité d'utiliser le glycogène comme source de carbone, qui est un substrat endogène secrété par la muqueuse vaginale. Cette souche présente par ailleurs la meilleure croissance en présence de bile.

Les résultats obtenus sont les suivants :

**Tableau 1 - Dernier test de sélection de la souche IP174178**

| Souche de référence | Taux d'adhésion aux cellules épithéliales | Croissance en présence de glycogène | Croissance en présence de bile |
|---|---|---|---|
| PB 0040 | 37% | - | + |
| IP 174178 | 58% | ++ | ++ |
| PB 0042 | 62% | - | + |

### Exemple 2- Profil biochimique

Les profils biochimiques sont obtenus à partir de l'équipement VITEK II compact (Biomérieux) qui est un automate pour l'identification bactérienne par comparaison des capacités de fermentation de sucres spécifiques. Nous obtenons le profil biochimique suivant : 11033303414010.

**Tableau 2. Profil biochimique de la souche IP174178**

| Détails biochimiques | | | | | | | | | | | | | | | | | |
|---|---|---|---|---|---|---|---|---|---|---|---|---|---|---|---|---|---|
| 2 | APPA | + | 4 | dGAL | - | 5 | ODC | - | 6 | PheA | + | 7 | ARG | - | 8 | PVATE | - |
| 9 | BGAL | - | 10 | PYRA | - | 11 | SUCT | - | 12 | TyrA | + | 13 | dGLU | + | 17 | BGLU | |
| 18 | dMAL | + | 19 | dMAN | + | 21 | BXYL | - | 22 | O/129 R | + | 23 | ProA | + | 26 | LIP | - |
| 27 | AMAN | - | 30 | dMLZ | - | 31 | URE | - | 33 | SAC | + | 35 | dTRE | + | 36 | CIT | |
| 37 | BGURi | - | 40 | ILATk | - | 41 | AGLU | + | 43 | dSOR | + | 44 | AGAL | - | 46 | GlyA | - |
| 47 | dMLT | - | 50 | dRIB | - | 51 | MTE | + | 52 | IGLM | - | 53 | OPS | (+) | 54 | BdFUC | - |
| 56 | CMT | + | 59 | 2KG | - | 61 | ESC | - | 62 | ELLM | - | 64 | dXYL | - | | | |

### Exemple 3- Profil génotypique

Le profil génotypique de la souche *IP174178* a été établi sur l'équipement DIVERSILAB (Biomérieux). Il correspond à l'amplification de l'ADN au sein de séquences répétitives non codantes du génome. La disposition et la taille de ces fragments sont uniques pour chaque souche et permettent ainsi d'établir des profils uniques pour chacune d'entre elles : voir figure 1.

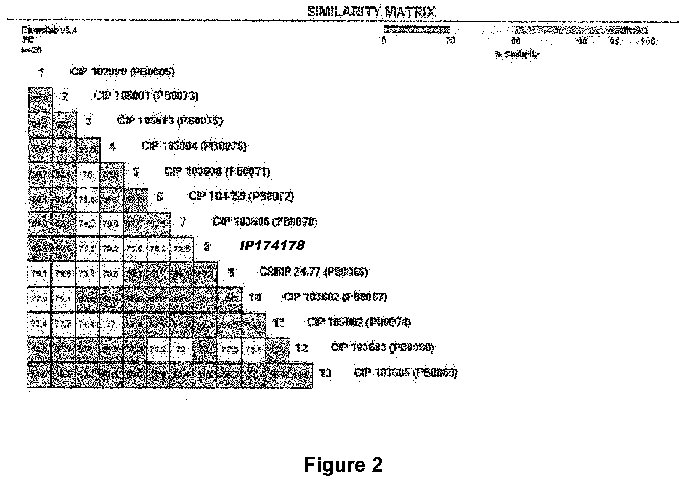

Nous avons alors comparé ce profil rep-PCR à celui de 26 souches de *Lactobacillus crispatus* dont 11 souches conservées à l'Institut Pasteur afin de s'assurer du caractère unique de la souche (cf. figure 2). La souche *IP174178* comporte à chaque fois des différences fortes, preuve de son unicité.

De plus, la souche *IP174178* est capable de croître sur du glycogène seul, ce qui présente un avantage pour son implantation dans la muqueuse vaginale, et présente un fort taux de production d'H₂O₂ et d'acide lactique.

### Exemple 4- Séquençage et identification d'oligonucléotides spécifiques

Le séquençage de la souche *IP174178* a été réalisé par la technique 454 ROCHE. Suite à ce travail, une annotation a été réalisée. Au final, 1982 gènes codant pour des protéines ont été identifiés.

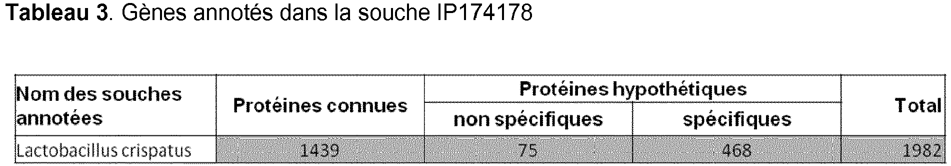

L'opération de séquençage a permis de mener un travail complémentaire de biologie moléculaire sur la souche afin de lui définir des couples d'amorces PCR spécifiques.

Au final trois amorces PCR ont été retenues et sont spécifiques à la souche lors d'une amplification comprenant 30 cycles. Ces amorces permettent d'identifier aisément et de façon très spécifique la souche de *L. crispatus IP174178* dans un échantillon biologique, par exemple.

**Tableau 4. Séquences des amorces PCR spécifiques de la souche IP174178**

| Nom | Numéro de séquence | Séquence | sens |
|---|---|---|---|
| Contig00070 | SEQ ID N°2 | CAACAGGCATCCCTAAGTCT | sens |
| | SEQ ID N°3 | CAACGTCTTCAGACCACATC | anti sens |
| Contig00074 | SEQ ID N°4 | TCTGCCTCATGTAGATCCAA | sens |
| | SEQ ID N°5 | GCTCCATCTTTAAGACCCAA | anti sens |
| Contig00145 | SEQ ID N°6 | AAGTGTTATGGGCCTAGTCG | sens |
| | SEQ ID N°7 | GCCACCACTTTTGCTTTAAT | anti sens |

### Exemple 5- Préparation de la « formulation A »

La souche *IP174178* est cultivée dans le milieu de culture suivant :
116 g/l de lait, 15 g/l de dextrose, 10 g/l d'autolysat de levure, 2 ml/l de Tween.

Après inoculation du milieu de culture, la fermentation est maintenue à une température de 37°C par un dispositif thermostaté approprié pendant 48 à 72 heures. En fin de fermentation, la culture, ayant atteint une numération supérieure ou égale à 10⁸ UFC/ml, est versée dans une cuve de mélange. La culture est agitée fortement pour briser le caillé et les adjuvants de lyophilisation suivants sont ajoutés: 110 g/l de lait, 101,5 g/l FOS, 9,5 ml/l glutamate de sodium, 5,25 g/l acide ascorbique, 113 g/l thiosulfate de sodium.

La lyophilisation est ensuite réalisée selon les conditions classiques bien connues de l'homme du métier : les bactéries sont réparties aseptiquement dans des plateaux en acier inoxydable, sont congelées rapidement à -40°C, puis soumises à une opération de sublimation à -22°C. Un étuvage à 37°C est ensuite réalisé.

Le produit résultant est ensuite broyé et tamisé sous atmosphère contrôlée. La poudre obtenue peut être avantageusement mélangée avec 1% de stéarate de magnésium afin de faciliter le remplissage des capsules / gélules.

La poudre ainsi obtenue est répartie dans des gélules de taille n° 0 à raison de 350 mg de poudre par gélule.

### Exemple 6 - Caractéristiques fonctionnelles de la souche IP174178 et de la 'formulation A'

La souche identifiée *IP174178* seule ou dans sa 'formulation A' telle que présentée dans l'exemple 5 ont été testées en parallèle pour déterminer leurs caractéristiques fonctionnelles.

Dans la suite des exemples, on entend par 'souche pure' ou 'souche seule' ou 'souche IP174178' la souche IP174178 sans aucun adjuvant, et notamment sans les composés de la Formulation A, issue de colonies isolées sur gélose après conservation en cryotube dans un milieu de conservation classique.

### Activités antimicrobiennes

### Mise en évidence de la production de peroxyde d'hydrogène :

Les bactéries lactiques sont mises en culture dans un milieu MRS gélosé additionné de vitamine B12, de peroxydase et de tétraméthylbenzidine. Après incubation pendant 48h en anaérobiose, les boîtes sont mises en contact avec l'oxygène ambiant et les colonies produisant du peroxyde d'hydrogène bleuissent rapidement. Une échelle arbitraire de 0 à 5, permet de noter l'intensité de la production d'H₂O₂.

**Tableau 5. Caractéristiques fonctionnelles de la souche IP174178**

| | **Production H₂O₂** | **Production d'acide lactique en g/litre** | |
|---|---|---|---|
| | | **Glucose source** | **Glycogen source** |
| Formulation A | 5 | D-LACT: 3,69 | D-LACT: 3,91 |
| | | L-LACT: 4,88 | L-LACT: 4,74 |
| Souche IP174178 | 5 | D-LACT: 5,90 | D-LACT: 5,65 |
| | | L-LACT: 3,10 | L-LACT: 3,16 |

Les résultats rapportés ci-dessus illustrent que :
- La production de peroxyde d'hydrogène est maximale selon l'échelle arbitraire utilisée ;
- La souche utilise de façon quasi-équivalente, aussi bien avec du glucose que du glycogène, les voies métaboliques permettant la synthèse des D-et L-lactates. Or le glycogène étant une des principales sources de carbone en milieu vaginal, cette souche possède les propriétés nécessaires à la production d'acide lactique *in vivo.*

### Inhibition de la croissance de C. albicans et de G. vaginalis

**Objectif :** Evaluer la capacité de souches de lactobacilles à inhiber la croissance d'une souche pathogène : *Candida albicans ;* le même protocole est appliqué pour *G*. *vaginalis,* adapté à ce pathogène.

### Descriptif du protocole :

Suivre la croissance des pathogènes seuls et en présence d'une culture d'une souche de lactobacille avec détermination du titre de T0 à T28h.

Milieux de culture utilisés pour préparation des inocula et la lecture des titres :
- bouillon Sabouraud et gélose Sabouraud pour *Candida albicans* (à 37°C)
- bouillon/gélose de MRS pour les souches de Lactobacilles.

Le bouillon de culture de l'essai est un mélange homogène du milieu de culture du pathogène et du milieu de culture du probiotique.

### Préparation de l'essai :

Préparation des inocula :
- ***Candida albicans:*** culture sur bouillon Sabouraud - 0,3 ml dans 30 ml à 37°C pendant 48h.
- ***Souche probiotique :***
   - **Formulation A :** on place 0,35g de poudre de Formulation A dans 10 ml de bouillon MRS - à l'étuve à 37°C pendant 48h.
   - **souche pure *IP174178* :** 0,2 ml de souche pure, issue de colonies isolées sur gélose, préalablement conservée en cryotube dans un milieu de conservation, est placée dans 10 ml de bouillon MRS - à l'étuve à 37°C pendant 48h.

Préparation du témoin Candida :
Pour la fabrication du témoin *Candida,* on place 5 ml de bouillon Sabouraud inoculé avec la souche pathogène (titre proche de 1,108 UFC/ml) et 5 ml de bouillon MRS non inoculé.

Mise en contact des inocula :
Placer 5 ml de la souche pathogène avec 5 ml de la souche selon l'invention dans ces différentes configurations.

On réalise les prélèvements et les déterminations du titre à T0, T4h, T24h et T28h. Les résultats sont exprimés en UFC / ml. La concentration en UFC à T0 n'est certes pas la même dans les trois cas comparés (cela tient aux conditions de travail) mais les valeurs au T0 sont du même ordre de grandeur, de sorte que la différence est sans effet sur le résultat.

**Tableau 6A. Inhibition Candida albicans**

| | **Temps de contact en heures** | | | |
|---|---|---|---|---|
| | **0** | **T4h** | **T24h** | **T28h** |
| Souche pure IP174178 | 1,24. 10⁷ | 7,87. 10⁶ | 1,40. 10⁶ | 4,60. 10⁵ |
| Formulation A | 5,43. 10⁶ | 5,47. 10⁶ | 0 | 0 |
| Témoin *Candida albicans* | 6,20. 10⁶ | 9,72. 10⁶ | 1,72. 10⁷ | 1,40. 10⁷ |

**Tableau 6B. Inhibition Gardnerella vaginalis**

| | **Temps de contact en heures** | | | |
|---|---|---|---|---|
| | **0** | **T4h** | **T24h** | **T28h** |
| Souche pure IP174178 | 1,53. 10⁷ | 1,67. 10⁶ | 0 | 0 |
| Formulation A | 3,87. 10⁶ | 6,73. 10¹ | 0 | 0 |
| Témoin *Gardnerella vaginalis* | 6,33. 10⁷ | 3,83. 10⁷ | 6,71. 10⁷ | 5,05. 10⁷ |

**Tableau 6C- Inhibition Candida albicans - influence de la concentration en thiosulfate de sodium dans la formulation de la souche IP174-178**

| | | | |
|---|---|---|---|
| Il a été préparé une Formulation A1 et une Formulation A2 en procédant comme pour la Formulation A, à cela près que la Formulation A1 ne contient pas de thiosulfate de sodium et que la Formulation A2 n'en contient que 50 g/l. L'inhibition de la croissance de *C. albicans* a été mesurée dans les trois cas. | | | |

| | **Diminution en log₁₀** | | |
|---|---|---|---|
| | **4h** | **24h** | **28h** |
| *Formulation A1 (0 g*/*l thiosulfate)* | 0 | 1 | 2 |
| *Formulation A2 (50 g*/*l thiosulfate)* | 0 | 3 | 3 |
| *Formulation A (113 g*/*l thiosulfate)* | 0 | 6 | 7 |

Dans ce tableau, les résultats sont exprimés comme la diminution logarithmique décimale (log10) du nombre d'UFC entre la numération à T0 et aux différents points de prélèvement (T4h, T24h et T28h).

Les résultats présentés ci-dessus, dans les Tableaux 6A-6C, indiquent que :
- L'inhibition de la croissance du pathogène *C*. *albicans* est réelle dès 24h, que ce soit par la souche pure ou par la souche en Formulation A. Cependant seule la Formulation A permet d'atteindre une inhibition complète au bout de 24 heures. ;
- L'inhibition de la croissance du pathogène *G. vaginalis* est complète au bout de 24 heures de co-culture, en présence de la souche seule ou dans sa Formulation A ; l'inhibition de la croissance du pathogène est néanmoins plus rapide avec la souche dans sa Formulation A (dès 4h).
- La présence de thiosulfate de sodium dans la formulation A permet de potentialiser l'effet anti-pathogène de la souche *IP 174178.* En effet, plus la concentration de thiosulfate de sodium est importante, plus l'inhibition observée à 24h est forte.

### Résistances gastriques

Suivant les individus et surtout le moment de la journée, le pH gastrique varie entre 2,5 et 5. La résistance de la souche IP174178, seule ou dans la formulation A, a été testée dans des milieux à pH=2,5, 3 et 4. Les résultats sont exprimés en diminution de la quantité de bactéries, au cours du temps.

**Tableau 7. Résistance de la souche seule ou dans sa formulation A, à des pH faibles**

| | **Résistance à pH 2,5 Réduction en Log10** | | **Résistance à pH 3 Réduction en Log10** | | **Résistance à pH 4 Réduction en Log10** | |
|---|---|---|---|---|---|---|
| | **45 min** | **90 min** | **45 min** | **90 min** | **45 min** | **90 min** |
| Formulation A | -1,12 log | -4,36 log | -0,18 log | -0,72 log | -0,02 log | -0,15 log |
| Souche IP174178 | -0,73 log | -2,59 log | -0,51 log | -0,58 log | -0,68 log | -0,80 log |

Les résultats montrent qu'au bout de 90 minutes, à pH=3 ou 4, la perte de bactéries est faible, de moins d'un logarithme 10. La résistance à pH=2,5 est plus faible, notamment lorsque la souche est utilisée dans sa formulation A.

La résistance à la bile a également été testée, au cours du temps (60, 120, 180 et 240 minutes), en présence de 3% de bile. Les résultats sont exprimés en comparant la quantité de bactéries survivantes dans le milieu témoin sans bile, et dans le milieu comportant 3% de bile.

**Tableau 8. Résistance de la souche seule ou dans sa formulation A, à la présence de bile**

| **Présence de 3% de bile dans le milieu de culture** | **Comparaison milieu sans bile/milieu avec bile en log décimale** | | | | |
|---|---|---|---|---|---|
| | **0** | **60 mn** | **120 mn** | **180 mn** | **240 mn** |
| Souche pure IP174178 | -0,46 | -0,50 | -0,54 | -0,63 | -0,86 |
| Formulation A | -0,56 | -0,66 | -0,58 | -0,68 | -0,79 |

Au bout de 240 minutes, soit 4 heures, la différence entre le témoin cultivé sans bile et le milieu avec bile est de moins d'un logarithme décimal, ce qui signifie que la croissance de la bactérie IP174178 est peu affectée par la présence de bile.

### Résistance aux antibiotiques

**Tableau 9. Résistance aux antibiotiques de la souche seule ou dans sa formulation A**

| **Antibiotiques testés** | **Formulation A** | **Souche pure** IP174178 | **Antibiotiques testés** | **Formulation A** | **Souche pure** IP174178 |
|---|---|---|---|---|---|
| **Tétracycline** | **Sensible** | **Sensible** | **Acide nalidixique** | Résistant | Résistant |
| **Colistine** | Résistant | Résistant | **Erythromycine** | **Sensible** | **Sensible** |
| **Acide fusidique** | Résistant | Résistant | **Polymyxine** | Résistant | Résistant |
| **Chloramphénicol** | **Sensible** | **Sensible** | **Acide pipémidique** | Résistant | Résistant |
| **Streptomycine** | Résistant | Résistant | **Clindamycine 25µg** | **Sensible** | **Sensible** |
| **Triméthoprim sulfaméthoxazole** | Résistant | Résistant | **Clindamycine 2µg** | **Sensible** | **Sensible** |
| **Métronidazole 50µg** | Résistant | Résistant | | | |

Les gènes suivants, connus pour être impliqués dans la résistance aux antibiotiques listés dans la tableau 10 ci-dessous, ont été identifiés comme étant exprimés dans la souche *L*. *crispatus* :

| **Antibiotiques** | | **L. crispatus IP174178** |
|---|---|---|
| **Famille** | **Nom** | |
| Aminoglycoside | Gentamycine, | *aac3* |
| | Neomycine | |
| | Kanamycine | |
| | Streptomycine | |
| Diaminopyrimidine | Trimethoprime | *dfrA* |
| Fusidanine | Acide fusidique | *fusA* |
| Quinolone | Acide nalidixique | *gyrA,norA* |
| Fluoroquinolone | Ciprofloxacine | *gyrA,norA* |

### Adhésion aux cellules épithéliales vaginales CRL-2616

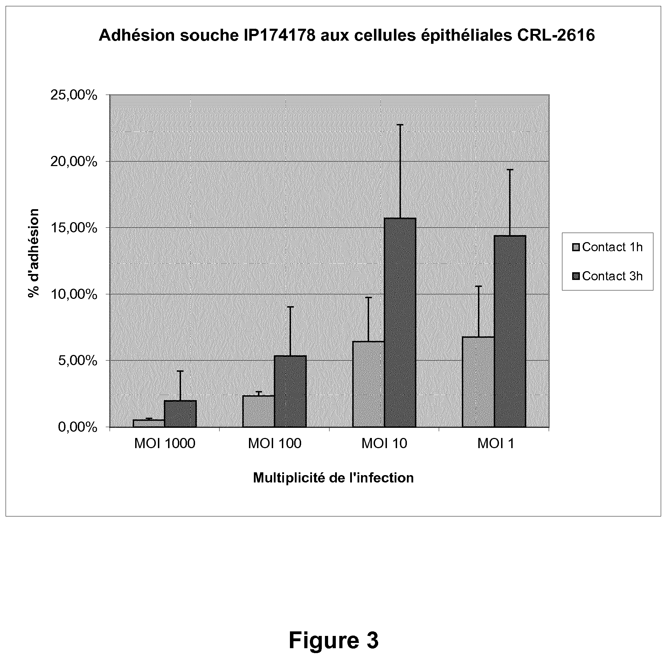

Les résultats sont présentés en figure 3, et ne concernent que la souche seule *IP174178.*

Au cours du temps, le pourcentage d'adhésion des bactéries IP174178 aux cellules CRL-2616 augmente et atteint 15% d'adhésion au bout de 3 heures, lorsque la multiplicité de l'infection est de 10 ou 1.

### REFERENCES

### DOCUMENTS BREVETS

WO 84/04675
WO 2000/035465
WO 2006/045475
US 2002/0044926
US 6.093.394
US 6.468.526
US 7.807.440
US 2010/0151026
EP 0 949 330
EP 1 824 500
EP 1 812 023
EP 1 427 808
EP 1 011 721

### DOCUMENTS de LITTERATURE SCIENTIFIQUE

Martin R, Soberôn N, Vaneechoutte M, Flórez AB, Vázquez F, Suárez JE. Characterization of indigenous vaginal lactobacilli from healthy women as probiotic candidates. Int Microbiol. 2008 Dec;11 (4):261-6.
Antonio, MAD, SE Hawes, and SL Hillier. The identification of vaginal Lactobacillus species and the demographic and microbiologic characteristics of women colonized by these species. J. Infect. Dis. (1999) 180: 1950-1956.
Man J.C., de Rogosa M. and Sharpe, ME. A medium for the cultivation of Lactobacilli, Appl. Bact. (1960) 23, 130-135

## Revendications

1. Souche isolée de *Lactobacillus crispatus,* identifiée *IP174178 et* déposée à la CNCM sous le numéro d'accès I-4646.

2. Composition pharmaceutique comprenant une souche selon la revendication 1.

3. Composition pharmaceutique selon la revendication 2, comprenant au moins 10⁹ CFU de ladite souche.

4. Composition pharmaceutique selon l'une des revendications 2 ou 3, comprenant, en outre, une matrice de conservation et optionnellement d'autres principes actifs.

5. Composition pharmaceutique selon l'une quelconque des revendications 2 à 4, comprenant, en outre, du thiosulfate de sodium.

6. Composition pharmaceutique selon l'une quelconque des revendications 2 à 5, formulée pour une administration par voie vaginale ou par voie orale.

7. Composition selon l'une quelconque des revendications 2 à 6, pour son utilisation dans le traitement et la prévention d'infections génitales et urogénitales telles que les vaginoses et les candidoses.

8. Dispositif médical, notamment tampon vaginal, renfermant une composition selon l'une quelconque des revendications 2 à 6, pour son utilisation dans le traitement et la prévention d'infections génitales et urogénitales telles que les vaginoses et les candidoses.

9. Composition nutraceutique comprenant une souche selon la revendication 1.

10. Composition pharmaceutique selon l'une quelconque des revendications 2 à 6, pour son utilisation dans le maintien ou la restauration d'une flore vaginale bénéfique.

## Patentansprüche

1. Isolierter Stamm von *Lactobacillus crispatus,* identifiziert *IP174178* und bei der CNCM unter der Zugangsnummer 1-4646 hinterlegt.

2. Pharmazeutische Zusammensetzung, umfassend einen Stamm nach Anspruch 1.

3. Pharmazeutische Zusammensetzung nach Anspruch 2, umfassend mindestens 10⁹ KBE des Stamms.

4. Pharmazeutische Zusammensetzung nach einem der Ansprüche 2 oder 3, umfassend ferner eine Konservierungsmatrix und optional weitere Wirkprinzipien.

5. Pharmazeutische Zusammensetzung nach einem der Ansprüche 2 bis 4, umfassend ferner Natriumthiosulfat.

6. Pharmazeutische Zusammensetzung nach einem der Ansprüche 2 bis 5, formuliert für eine Verabreichung auf vaginalem Weg oder auf oralem Weg.

7. Zusammensetzung nach einem der Ansprüche 2 bis 6 zur Verwendung bei der Behandlung und Vorbeugung von Genital- und Urogenitalinfektionen wie Vaginosen und Candidosen.

8. Medizinische Vorrichtung, insbesondere Vaginaltampon, enthaltend eine Zusammensetzung nach einem der Ansprüche 2 bis 6 zur Verwendung bei der Behandlung und Vorbeugung von Genital- und Urogenitalinfektionen wie Vaginosen und Candidosen.

9. Nutrazeutische Zusammensetzung, umfassend einen Stamm nach Anspruch 1.

10. Pharmazeutische Zusammensetzung nach einem der Ansprüche 2 bis 6 zur Verwendung bei der Erhaltung oder bei der Wiederherstellung einer günstigen Vaginalflora.

## Claims

1. An isolated strain of *Lactobacillus crispatus,* identified as *IP174178* and deposited at the CNCM under the accession number 1-4646.

2. A pharmaceutical composition including a strain according to claim 1.

3. The pharmaceutical composition according to claim 2, including at least 10⁹ CFU of said strain.

4. The pharmaceutical composition according to one of claims 2 or 3, further including a preservation matrix and optionally other active ingredients.

5. The pharmaceutical composition according to any one of claims 2 to 4, further including sodium thiosulfate.

6. The pharmaceutical composition according to any one of claims 2 to 5, formulated for vaginal administration or for oral administration.

7. The composition according to any one of claims 2 to 6, for use in the treatment and prevention of genital and urogenital infections such as vaginosis or candidiasis.

8. A medical device, in particular a vaginal tampon, containing a composition according to any one of claims 2 to 6, for use in the treatment and prevention of genital and urogenital infections such as vaginosis or candidiasis.

9. A nutraceutical composition including a strain according to claim 1.

10. The pharmaceutical composition according to any one of claims 2 to 6, for use in maintaining or restoring beneficial vaginal flora.


### SEQUENCE LISTING

<110> SPMD
<120> Nouvelle souche de lactobacillus crispatus
<130> D30896
<150> FR 1256569
   <151> 2012-07-09
<160> 7
<170> PatentIn version 3.5
<210> 1
   <211> 1061
   <212> DNA
   <213> Lactobacillus crispatus
<400> 1
   agccatgcac cacctgtctt agcgtccccg aagggaactt tgtatctcta caaatggcac 60
   tagatgtcaa gacctggtaa ggttcttcgc gttgcttcga attaaaccac atgctccacc 120
   gcttgtgcgg gcccccgtca attcctttga gtttcaacct tgcggtcgta ctccccaggc 180
   ggagtgctta atgcgttagc tgcagcactg agaggcggaa acctcccaac acttagcact 240
   catcgtttac ggcatggact accagggtat ctaatcctgt tcgctaccca tgctttcgag 300
   cctcagcgtc agttgcagac cagagagccg ccttcgccac tggtgttctt ccatatatct 360
   acgcattcca ccgctacaca tggagttcca ctctcctctt ctgcactcaa gaaaaacagt 420
   ttccgatgca gttcctcggt taagccgagg gctttcacat cagacttatt cttccgcctg 480
   cgctcgcttt acgcccaata aatccggaca acgcttgcca cctacgtatt accgcggctg 540
   ctggcacgta gttagccgtg actttctggt tgattaccgt caaataaagg ccagttacta 600
   cctctatcct tcttcaccaa caacagagct ttacgatccg aaaaccttct tcactcacgc 660
   ggcgttgctc catcagactt gcgtccattg tggaagattc cctactgctg cctcccgtag 720
   gagtttgggc cgtgtctcag tcccaatgtg gccgatcagt ctctcaactc ggctatgcat 780
   catcgccttg gtaagccttt accttaccaa ctagctaatg caccgcgggg ccatcccata 840
   gcgacagctt acgccgcctt ttaaaagctg atcatgcgat ctgctttctt atccggtatt 900
   agcacctgtt tccaagtggt atcccagact atggggcagg ttccccacgt gttactcacc 960
   catccgccgc tcgctttcct aacgtcatta ccgaagtaaa tctgttagtt ccgctcgctc 1020
   gacttgcatg tattaggcac gccgccagcg ttcgtcctga g 1061
<210> 2
   <211> 20
   <212> DNA
   <213> Artificial Sequence
<220>
   <223> Oligonucleotide
<400> 2
   caacaggcat ccctaagtct 20
<210> 3
   <211> 20
   <212> DNA
   <213> Artificial Sequence
<220>
   <223> Oligonucleotide
<400> 3
   caacgtcttc agaccacatc 20
<210> 4
   <211> 20
   <212> DNA
   <213> Artificial Sequence
<220>
   <223> Oligonucleotide
<400> 4
   tctgcctcat gtagatccaa 20
<210> 5
   <211> 20
   <212> DNA
   <213> Artificial Sequence
<220>
   <223> Oligonucleotide
<400> 5
   gctccatctt taagacccaa 20
<210> 6
   <211> 20
   <212> DNA
   <213> Artificial Sequence
<220>
   <223> Oligonucleotide
<400> 6
   aagtgttatg ggcctagtcg 20
<210> 7
   <211> 20
   <212> DNA
   <213> Artificial Sequence
<220>
   <223> Oligonucleotide
<400> 7
   gccaccactt ttgctttaat 20